(19) Europäisches Patentamt
European Patent Office
Office européen des brevets

(11) Publication number: **0 014 475**
**B1**

(12) **EUROPEAN PATENT SPECIFICATION**

(45) Date of publication of patent specification: **15.02.84**

(21) Application number: **80100663.6**

(22) Date of filing: **08.02.80**

(51) Int. Cl.³: **C 07 D 471/04,**
C 12 P 17/18,
A 61 K 31/435 //C12N9/80,
(C07D471/04, 221/00,
205/00)

(54) **Optically active cephalosporin analogs, process for their preparation and their use in the preparation of pharmaceutical compositions.**

(30) Priority: **10.02.79 JP 14533/79**
**24.08.79 JP 107070/79**
**14.11.79 JP 146488/79**

(43) Date of publication of application:
**20.08.80 Bulletin 80/17**

(45) Publication of the grant of the patent:
**15.02.84 Bulletin 84/7**

(84) Designated Contracting States:
**BE CH DE FR GB IT NL SE**

(56) References cited:
**DE - A - 2 714 880**
**GB - A - 2 017 102**

**CEPHALOSPORINS and Penicillins, pages 13 and 540**

**BURGER'S Medicinal Chemistry, 3rd Edition, pages 81-87**

**The file contains technical information submitted after the application was filed and not included in this specification**

(73) Proprietor: **KYOWA HAKKO KOGYO CC., LTD**
**Ohtemachi Bldg. Ohtemachi 1-chome Chiyoda-ku**
**Tokyo (JP)**

(72) Inventor: **Hirata, Tadashi**
**1566-315, Nara-machi, Midori-ku**
**Yokohama-shi Kanagawa-ken (JP)**
Inventor: **Hashimoto, Yukio**
**3-5-15, Chuorinkan**
**Yamato-shi Kanagawa-ken (JP)**
Inventor: **Ogasa, Takehiro**
**3-6-6, Asahi-machi**
**Machida-shi Tokyo (JP)**
Inventor: **Kobayashi, Shigeru**
**71-9, Kiso-machi**
**Machida-shi Tokyo (JP)**
Inventor: **Matsukuma, Ikuo**
**2273-1, Ikuwa-cho**
**Yokkaichi-shi Mie-ken (JP)**
Inventor: **Kimura, Kazuo**
**2-1-5, Kyowa-cho**
**Hofu-shi Yamaguchi-ken (JP)**
Inventor: **Yoshiie, Shigeo**
**2-14-10, Asahi-machi**
**Machida-shi Tokyo (JP)**
Inventor: **Takasawa, Seigo**
**125-5, Minamiyana**
**Hadano-shi Kanagawa-ken (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 014 475 B1

Courier Press, Leamington Spa, England.

(74) Representative: **Vossius Vossius Tauchner Heunemann Rauh**
**Siebertstrasse 4 P.O. Box 86 07 67**
**D-8000 München 86 (DE)**

# Optically active cephalosporin analogs, process for their preparation and their use in the preparation of pharmaceutical compositions.

## Background of the Invention

The present invention relates to optically active cephalosporin analogs and, more particularly, it pertains to optically active compounds of cephalosporin analogs represented by the general formula (I)

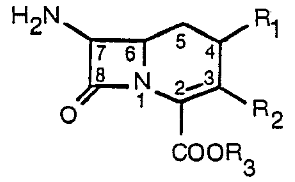

(I)

wherein $R_1$ represents a hydrogen or a straight or branched alkyl group having 1 to 5 carbon atoms, $R_2$ represents a hydrogen or a halogen atom, $R_3$ represents a hydrogen or protective group of carboxylic acid, and the hydrogens at the 6- and 7-positions have cis configuration, the pharmaceutically acceptable salts thereof and processes for producing the same.

Heretofore, a carbacephem compound, which is named according to the nomenclature in J. Am. Chem. Soc. *96,* 7584 (1974), wherein the sulfur atom of cephalosporin is substituted with a carbon atom and which has a substituted methyl group at the 3-position is described in the above reference and J. Med. Chem. *20,* 551 (1977). However, no compound of this type having especially strong antibacterial activity has been reported.

The present inventors have succeeded in preparing carbacephem compounds having various substituents at the 4-, 5- and 3-positions [The numbering system is as shown in general formula (1)]. These compounds are described in the specifications of Japanese Patent Applications No. 34696/78 (Japanese Published Unexamined Patent Application No. 128591/79) DE-A-2911786, 122403/78, 133072/78, 162005/78 and 8408/79.

Further, the present inventors have succeeded in preparing novel acylated carbacephems which are new antibiotics having strong antibacterial activities. These are described in Japanese Patent Applications No. 34696/78 (Japanese Published Unexamined Patent Application No. 128591/79), 122402/78, 127027/78, 133071/78, 162006/78, 162007/78, 162008/78, 8409/79 and DE-A-2911787.

However, the cephalosporin analogs mentioned above are prepared by synthetic methods using optically inactive starting compounds, and they are optically inactive dl [represented by (±)] compounds unless they have optically active acyl group. More specifically, compounds represented by the general formula (I) wherein the hydrogen atoms at the 6- and 7-positions have cis configuration are present as a mixture of equal amounts of the mirror image compounds represented by the formulae (I—1) and (I—2)

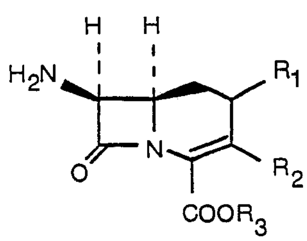

(I—1)

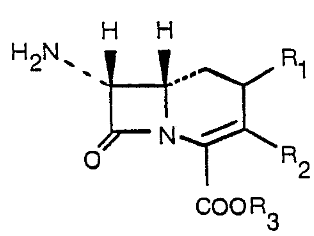

(I—2)

wherein $R_1$, $R_2$ and $R_3$ have the same significance as defined above. However, no method of isolating one of these enantiomers has been reported.

To this end, it has now been found that one of the optically active mirror image compounds can be prepared and isolated.

## Summary of the Invention

In accordance with the present invention, optically active compounds are prepared of cephalosporin analogs represented by the formula:

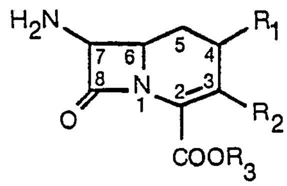

(I)

(wherein $R_1$ represents a hydrogen or a straight or branched alkyl group having 1 to 5 carbon atoms, $R_2$ represents a hydrogen or a halogen atom, $R_3$ represents a hydrogen or a protective group of carboxylic acid and the hydrogens at the 6- and 7-positions have cis configuration) and salts thereof.

In the foregoing general formula (I), when the group 7 is a straight or branched alkyl group having 1 to 5 carbon atoms it may be a group such as methyl group, ethyl group, n-propyl group, i-propyl group, n-butyl group, i-butyl group, sec-butyl group, or t-butyl group.

The ester group $COOR_3$ is a group readily convertible to COOH employed in the chemistry of penicillins and cephalosporins.

The group $R_3$, may be a straight-chain or branched alkyl group having 1 to 5 carbon atoms such as methyl group, ethyl group, n-propyl group, i-propyl group, n-butyl group, i-butyl group, sec-butyl group or t-butyl group, a straight-chain or branched alkoxymethyl group having 1 to 5 carbon atoms such as methoxymethyl group or exthoxymethyl group; a straight or branched halogenated alkyl group having 1 to 5 carbon atoms such as chloromethyl group, 2,2,2,-trichloroethyl group or 2,2,2-trifluoroethyl group; a lower alkylsulfonylethyl group such as methylsulfonylethyl group or ethylsulfonylethyl group, an arylmethyl group having 7 to 12 carbon atoms such as benzyl group, diphenylmethyl group, trityl group or triphenylmethyl group; a substituted arylmethyl group having 7 to 20 carbon atoms wherein the substituent is methoxy group, nitro group, and the number of the substituents on the phenyl ring is 1 to 5; or a protective group of carboxylic acid represented by the general formula (II)

$$-\underset{\displaystyle R_5}{\underset{|}{C}}HOCOR_4 \qquad (II)$$

wherein $R_4$ represents a straight-chain or branched lower alkyl group having 1 to 5 carbon atoms, a straight-chain or branched lower alkoxy group having 1 to 5 carbon atoms, or a phenyl group, and $R_5$ represents a hydrogen or a straight-chain or branched lower alkyl group having 1 to 5 carbon atoms. As the halogen atom, chlorine, bromine or iodine is exemplified.

The optically active compounds of cephalosporin analogs represented by the general formula (I), that is, one of the enantiomers, are prepared, according to the present invention, by an optically selective deacylation reaction using an enzyme and an optically inactive dl compound having an acyl group as a certain starting compound. The desired compound is obtained in a remarkably high yield by this method.

A compound wherein an optically active acyl group is introduced to a dl form of a compound represented by the general formula (I), referred to as Compound (I) hereinafter, that is, the compound represented by the general formula (A)

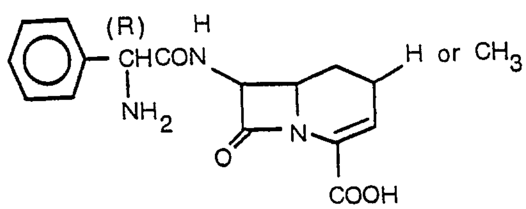

(A)

is separated to diastereoisomers (Japanese Patent Application No. 127027/78) (DE-A-2911787).

The optically active compounds obtained in the present invention are assumed to have the absolute structure represented by the general formula (I—1), that is (6R, 7S), from various properties, strong antimicrobial activity of the acyl compounds as compared with the corresponding optically inactive dl-compound and the relationship between the absolute chemical structure of cephalosporins and activities thereof. These compounds are particularly useful as intermediates in the preparation of optically active acylated compounds which are strong antibacterial agents.

In the following description, the optically active compounds are described with reference to the general formula (I—1). Additionally, the compounds in the following examples and reference examples are named according to the assumed absolute structural formula.

Detailed Description of the Invention

Optically active compounds of the cephalosporin analogs represented by the general formula (I) or

compounds represented by the assumed absolute structural formula (I—1) are produced by optically selective deacylation of a compound represented by the general formula (III)

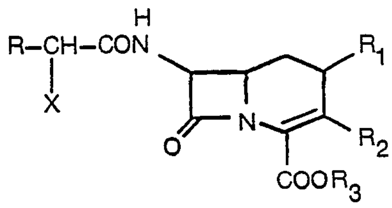

(III)

wherein R represents a substituted or unsubstituted unsaturated six-membered carbocyclic or five-membered heterocyclic group, wherein the substituent is represented by a hydroxy group, halogen atom, nitro group or methansulfonamide group, X represents a hydrogen, an amino group, a hydroxy group or an alkyl group having 1 to 5 carbon atoms (referred to as Compound [III] hereinafter), $R_1$, $R_2$ and $R_3$ have the same significance as defined above, and the hydrogens at the 6- and 7-positions have cis configuration.

As the unsaturated six-membered carbocyclic and five-membered heterocyclic group, phenyl group, cyclohexenyl group, cyclohexadienyl group, thienyl group, furyl group pyrrolyl group, thiazolyl group, iso-thiazolyl group, oxazolyl group, iso-oxazolyl group, imidazolyl group, pyrazolyl group, triazolyl group, tetrazolyl group, pyridinyl group and pyrazinyl group are exemplified. As the substituent, hydroxy group, halogens, nitro group or methanesulfonamide group are mentioned. As the lower alkyl group, straight-chain or branched alkyl groups having 1 to 5 carbon atoms such as methyl group, ethyl group, n-propyl group, i-propyl group, n-butyl group, i-butyl group, sec-butyl group or t-butyl group are mentioned.

The optically selective deacylation of Compound [III] to obtain optically active Compound [I] is carried out in the presence of an enzyme obtained from a microorganism capable of producing optically active Compound [I] by optically selective deacylation of Compound [III].

As the microorganism having an ability of optically selective deacylation, microorganisms belonging to the genus *Aeromonas, Achromobacter, Arthrobacter, Acetobacter, Alcaligenes, Escherichia, Xanthomonas, Kluyvera, Gluconobacter, Clostridium, Comamonas, Corynebacterium, Sarcina, Staphylococcus, Spirillium, Bacillus, Pseudomonas, Flavobacterium, Brevibacterium, Protaminobacter, Proteus, Beneckea, Micrococcus, Mycoplana* and *Rhodopseudomonas* are used.
The following strains are examples of the microorganism.

| | |
|---|---|
| *Aeromononas hydrophila* | IFO 12634 |
| *Achromobacter aceris* | IFO 3320 |
| *Arthrobacter simplex* | ATCC 15799 |
| *Acetobacter aurantius* | IFO 3245, IAM 1812 |
| *Acetobacter sp.* | ATCC 21760 |
| *Alcaligenes faecalis* | ATCC 8750 |
| *Escherichia coli* | ATCC 11105 |
| *Escherichia coli* | ATCC 13281 |
| *Xanthomonas citri* | IFO 3835 |
| *Xanthomonas physalidicola* | IFO 13555 |
| *Kluyvera citrophila* | ATCC 21285 |
| *Gluconobacter liquefaciens* | ATCC 14835 |
| *Clostridium acetobutylicum* | IFO 3346 |
| *Comamonas sp.* | FERM-P 2410 |
| *Corynebacterium tritici* | IFO 1216 |

| | |
|---|---|
| *Sarcina lutea* | ATCC 9341 |
| *Staphylococcus aureus* | IFO 3060 |
| *Spirillum methamorphum* | IFO 12012 |
| *Bacillus megaterium* | ATCC 14945 |
| *Pseudomonas melanogenum* | ATCC 17808 |
| *Pseudomonas aeruginosa* | IFO 3451 |
| *Flavobacterium sp.* | ATCC 21429 |
| *Brevibacterium cerinum* | ATCC 15112 |
| *Protaminobacter alboflavus* | IFO 13221 |
| *Proteus rettgeri* | ATCC 9250 |
| *Beneckea hyperoptica* | ATCC 15803 |
| *Micrococcus luteus* | AHU 1427 |
| *Mycoplana bullata* | IFO 13267 |
| *Mycoplana dimorpha* | IFO 13213 |
| *Rhodopseudomonas spheroides* | ATCC 21286 |

For carrying out the optically selective deacylation reaction, the enzyme may be provided, more specifically, in any of the following forms:

1. As the culture liquor of the microorganism or treated matter thereof;
2. As cell bodies recovered from the culture broth by centrifugation which may be washed with saline water (usually about 1%), buffer solution, or as a cell suspension;
3. As a disrupted cell suspension, i.e., a suspension of the cell bodies disrupted mechanically or chemically;
4. As a cell free extract, i.e., a liquid obtained by removing the disrupted cell bodies from the disrupted cell suspension; or
5. As a purified enzyme solution which is obtained by recovering the enzyme protein with ammonium sulfate from the cell free extract and subjecting the enzyme protein to gel filtration, ion-exchange cellulose column chromatography or ion-exchange Sephadex® column chromatography.

Cells or the purified enzyme immobilized by a conventional method may be used.

The reaction is carried out at a temperature of 0 to 40°C, preferably 15 to 35°C and at a pH of 5 to 8 in an inactive solvent which does not affect the reaction.

As the solvent, water is most preferably used.

In order to dissolve the substitute or cephalosporin analogs, organic solvents such as acetone, methanol, ethanol, N,N-dimethylformamide or dimethylsulfoxide may be used. It is effective to add phosphate buffer, Veronal buffer or citric acid buffer to control the pH in the reaction. Reaction time being influenced by the kind and concentration of enzymes, the kind and concentration of substrates, reaction temperature or reaction pH, is generally 30 minutes to 24 hours. It is most preferable to terminate the reaction when the reaction ratio reaches maximum.

The concentration of cells is preferably 1 to 50 mg by dry weight per 1 ml of the reaction solution. When a purified enzyme is used, it is appropriate to use the amount of the enzyme having the same activity as that of the dry cell. The substrate Compound [III] is used in an amount of 0.5 to 50 mg per 1 ml of the reaction solution.

In the event the microorganism utilized also produces an enzyme such as $\beta$-lactamase or esterase, which tend to prevent the desired reaction, such microorganisms can be mutated by known techniques to obtain a mutant strain which has a reduced productivity of the undesirable enzyme. Alternatively, inhibitors of such enzymes may be added in the reaction system to raise the reaction ratio.

After the completion of the reaction, isolation of the desired compound is carried out by a conventional method employed in the isolation and purification of organic compounds from culture liquors such as absorption using various carriers, ion-exchange chromatography, gel filtration or liquid-liquid extraction.

Among the compounds represented by the general formula (I), the optically active compounds of the cephalosporin analogs represented by the general formula (I—3)

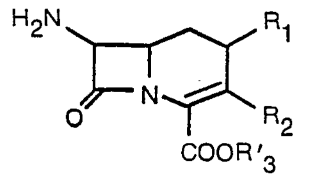

(I−3)

(wherein $R_1$ and $R_2$ have the same significance as defined above, $R'_3$ represents a protective group of carboxylic acid and the hydrogens at the 6- and 7-positions have cis configuration) may also be obtained by the esterification of the optically active cephalosporin analogs represented by the general formula (I—4)

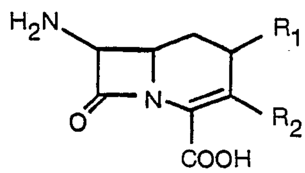

(I−4)

(wherein $R_1$ and $R_2$ have the same significance as defined above, and the hydrogens at the 6- and 7-positions have cis configuration) by a conventional method, that is, the compound represented by the formula (I—3′)

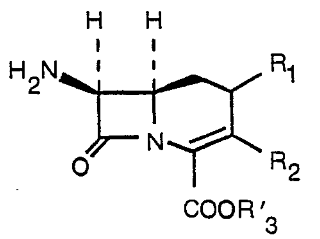

(I−3′)

(wherein $R_1$, $R_2$ and $R'_3$ have the same significance as defined above) are obtained by the esterification of the compound represented by the formula (I—4′)

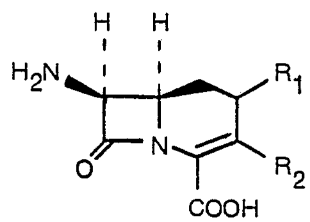

(I−4′)

(wherein $R_1$ and $R_2$ have the same significance as defined above) by a conventional method.

Optically active compounds of the present invention, that is, Compound [I—1], themselves are expected to have antimicrobial activities and the acyl compounds of the optically active Compound [I] (Compound [I—1]) have much stronger antimicrobial activities than the acyl compounds of the corresponding optically inactive Compound [I]. Examples of such compounds and antimicrobial activities thereof are described in Reference Examples.

The present invention is explained by the following Examples.

Example 1

Preparation of (+)-cis-7-amino-1-azabicyclo[4,2,0)]oct-2-en-8-on-2-carboxylic acid [(+)-cis-7-amino-2-carboxy-1-azabicyclo[4,2,0]oct-2-en-8-on]: (cis refers to the stereo-chemistry at 6- and 7-positions and the same shall apply hereinafter)

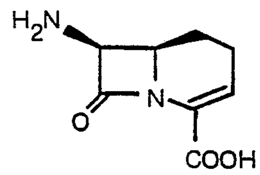

(+)

1—1. Preparation of disrupted cell suspension

1) Cultivation of a microorganism having an ability of optically selective deacylation.

As the seed strain, *Kluyvera citrophila* ATCC 21285 [Biological properties are described in J. General Applied Microbiology *3*, 28—31 (1957)] is used.

As the seed medium, an aqueous solution containing 1% polypeptone, 1% yeast extract, 0.5% meat extract, 0.5% sodium glutamate and 0.25% sodium chloride and adjusted at a pH of 7.0 with 5N-NaOH is used. One loopful of the seed strain is inoculated into 10 ml of the seed medium in a 50 ml test tube and culturing is carried out at a temperature of 30°C for 24 hours. The whole of the seed broth is inoculated into 300 ml of the culture medium in a 2 l Erlenmeyer flask and culturing is carried out at a temperature of 30°C with shaking. The composition of the main culture medium is the same as that of the seed medium.

2) Preparation of disrupted cell suspension

After culturing for 24 hours, the culture broth is subjected to centrifugation to obtain cell bodies. The cells are washed twice with 50 ml of 0.9% saline solution and suspended at a concentration of 40 mg/ml by dry weight in 1/30 M phosphate buffer solution. Then, 10 ml of the cell suspension is put in a 50 ml test tube and subjected to ultrasonic disintegration at 200 W for 2 minutes to obtain disrupted cell suspension. In the treatment, ultrasonic disintegrator Model UR200P (product of Tomy Seiko Co., Ltd.) is used.

1—2. Preparation of a substrate solution

In this step, 200 mg of (±)-cis-7-phenylacetamido-1-azabicyclo[4,2,0]oct-2-en-8-on-2-carboxylic acid obtained as in DE-A-2911787 is added to 9 ml of 1/30M phosphate buffer (pH 6.5). Since the compound is not dissolved, 2N-NaOH is added in a small portion and the mixture is again adjusted to a pH of 6.5 to dissolve the compound. Finally, deionized water is added to make 10 ml of a solution.

1—3. Enzyme reaction

In this step, 10 ml of the disrupted cell suspension mentioned above is added to 10 ml of the substrate solution and enzyme reaction is carried out at a temperature of 30°C for 80 minutes. Time course of the reaction is illustrated in Table 1.

Table 1

| Reaction period (minutes) | The amount of Compound [I—1] produced (mg/ml) | Yield (Mol ratio, %) |
|---|---|---|
| 10 | 2.0 | 33 |
| 20 | 2.6 | 43 |
| 40 | 2.9 | 48 |
| 60 | 3.0 | 50 |
| 80 | 3.0 | 50 |

As apparent from the Table 1, the reaction and yield are stationary after the conversion ratio of the mixture of the optically active isomers reaches 50% (mol ratio).

1—4. Isolation and Purification of the desired compound

After the completion of the reaction, cells are removed by centrifugation from the reaction solution. The supernatant is adjusted at a pH of 3.0 with 2N-hydrochloric acid and charged on a column (2.6 cm width, 51 cm height) packed with 270 ml of Diaion® HP—10 (product of Mitsubishi Kasei Co., Ltd.). Elution is carried out with deionized water and the eluate is collected in 5 ml fractions. The desired compound is eluted in fractions from 280 ml to 315 ml. The fractions are concentrated under reduced pressure, lyophilized and dissolved in a small amount of a mixture of water and methanol (50:50 by volume, the same shall apply hereinafter). The solution is charged on a column (1.6 cm width, 64.5 cm height) packed with 130 ml of Sephadex LH—20 (Farmacia Fine Chemicals Co., Ltd.). Elution is carried out with a mixture of water and methanol (50:50). The eluate is collected in 5 ml

fractions. Fractions from 65 ml to 85 ml are combined and concentrated under reduced pressure to remove methanol. Then, the residue is lyophilized to obtain 48 mg of a white powder. Properties of the product are as follows.

IR(KBr)$\nu_{max}^{cm^{-1}}$ : 1800, 1790, 1775, 1640, 1620

NMR(100M $D_2O$-DSS)$\delta$: 6.46(1H,dd,J=3.5, 4.7Hz), 4.88(1H,d,J=5.2Hz), 4.06(1H,m), 2.5—1.5(4H,m)

It is revealed that the compound has one mole of hydrochloric acid and water. The properties of the compound coincide well with those of the corresponding dl-compound. The value of optical rotation is $[\alpha]_D^{15°} = +48°$ [c=0.5, in 1M phosphate buffer solution (pH 7.0)] which coincides well with the value in Example 2 below, $[\alpha]_D^{15°} = 48.5°$ [c=0.5, in 1M phosphate buffer solution (pH 7.0)].

The compound shows a ninhydrin positive single spot at an Rf value of 0.22 on silica gel thin layer chromatography [thin layer plate Merck Art 5721 (product of E. Merck & Co.], solvent for development, isopropanol : acetic acid : water = 4 : 1 : 1]. The Rf value coincides with that of the optically inactive dl-compound.

Example 2

Preparation of (+)-cis- 7 -amino-1-azabicyclo[4,2,0]oct-2-en-8-on-2-carboxylic acid (Alternative method).

2—1. Preparation of disrupted cell suspension

The same procedure as in Example 1—1 is repeated.

2—2. Preparation of a substrate solution

In this step, 100 mg of (+)-cis-7 -[(R)-2-phenyl-2-aminoacetamido]-1-azabicyclo[4,2,0]oct-2-en-8-on-2-carboxylic acid obtained as in DE-A-2911787 is dissolved in 5 ml of 1/30M phosphate buffer solution (pH 6.5).

2—3. Enzyme reaction

In this step, 5 ml of the disrupted cell suspension mentioned above are added to 5 ml of the substrate solution and enzyme reaction is carried out at 30°C for 24 hours.

2—4. Isolation and Purification

46 mg of a white powder is obtained by a similar method as in Example 1—4. Properties of the compound coincide well with those of the compound obtained in Example 1.

$[\alpha]_D^{15°} = 48.5°$ [c=0.5, in 1M phosphate buffer solution .(pH 7.0)]

Example 3

Preparation of (—)-cis-7$\beta$-amino-4$\alpha$-methyl-1-azabicyclo[4,2,0]oct-2-en-8-on-2-carboxylic acid

3—1. Preparation of disrupted cell suspension

A similar procedure as in Example 1—1 is repeated.

3—2. Preparation of a substrate solution

A similar procedure as in Example 1—2 is repeated except that ($\pm$)-cis-7$\beta$-phenylacetamido-4$\alpha$-methyl-1-azabicyclo[4,2,0]oct-2-en-8-on-2-carboxylic acid obtained as in GO 2911787 is used.

3—3. Enzyme reaction

A similar procedure as in Example 1—3 is repeated except that the disrupted cell suspension and the substrate solution obtained in 3—1 and 3—2 are used. Reaction ratio becomes stationary in one hour. The reaction is continued for 120 minutes. The yield is 50% (mol ratio) of the ($\pm$) substrate.

3—4. Isolation and Purification of the desired compound

An almost similar procedure as in Example 1—4 is repeated. After the completion of reaction, cells are removed by centrifugation from the reaction solution. The supernatant is charged on a column (2.5 cm width, 46 cm height) packed with 220 ml of Diaion HP—10. Elution is carried out with deionized water and the eluate is collected in 5 ml fractions. The desired compound is eluted in fractions between 200 ml and 270 ml. The fractions are concentrated under reduced pressure, lyophilized, and dissolved in a small amount of water and methanol (50:50). The solution is charged on a column (1.6 cm width, 64.5 cm height) packed with 130 ml of Sephadex LH—20 and elution is carried out with a mixture of water and methanol (50:50). The eluate is collected in 5 ml of fractions. The fractions from 65 ml to 80 ml are combined and concentrated to remove methanol. Then, the residue is lyophilized to obtain 30.5 mg of a white powder. Properties of the compound are as follows.

IR(KBR)$\nu_{max}^{cm-1}$ : 1800, 1770(sh), 1760(sh), 1740, 1680, 1630
NMR(100M $D_2O$-DSS)$\delta$: 6.16(1H,d,J=5.1Hz), 4.52(1H,d,J=4.9Hz), 3.86(1H,m), 2.64(1H,m), 1.9—1.4(2H,m), 1.10(3H,d,J=7.3Hz)

It is revealed that the compound is a potassium salt having 2 moles of water. The properties above coincide well with those of the corresponding dl-compound. The compound shows a ninhydrin positive single spot at Rf=0.33 on a silica gel thin layer chromatography (the same silica gel as in Example 1—4 used). The Rf value coincides with that of the optically inactive dl-compound.

Optical rotation $[\alpha]_D^{15°} = -30°$ [c=0.5, in 1M phosphate buffer solution). The value coincides well with that in Example 4, $[\alpha]_D^{15°} = -30.8°$ [c=0.5, in 1M phosphate buffer solution (pH 7.0)].

Example 4

Preparation of (—)-cis-7$\beta$-amino-4$\alpha$-methyl-1-azabicyclo[4,2,0]oct-2-en-8-on-2-carboxylic acid (Alternative method)

4—1. Preparation of disrupted cell suspension

A similar procedure as in Example 3—1 is repeated.

4—2. Preparation of a substrate solution

100 mg of (+)-cis-7 -[(R)-2-phenyl-2-aminoacetamido] 4$\alpha$-methyl-1-azabicyclo[4,2,0]oct-2-en-8-on-2-carboxylic acid obtained as in DE-A-2911787 is dissolved in 5 ml of 1/30M phosphate buffer solution (pH 6.5).

4—3. Enzyme reaction

As in Example 1—3, 5 ml of the disrupted cell suspension described above is added to 5 ml of the substrate solution and enzyme reaction is carried out at a temperature of 30°C for 24 hours.

4—4. Isolation and Purification of the desired product

A similar procedure as in Example 3—4 is repeated to obtain 55 mg of a white powder. Properties of the compound coincide well with those in Example 3.

Optical rotation $[\alpha]_D^{15°} = -30.8°$ [c=0.5, in 1M phosphate buffer solution (pH 7.0)].

Example 5

Preparation of (+)-cis-7 -amino-1-azabicyclo[4,2,0]oct-2-en-8-on-2-carboxylic acid (Alternative method):

5—1. Cultivation of microorganisms

As the seed microorganism, the following strains are used.

| | |
|---|---|
| *Aeromononas hydrophila* | IFO 12634 |
| *Achromobacter aceris* | IFO 3320 |
| *Arthrobacter simplex* | ATCC 15799 |
| *Acetobacter aurantius* | IFO 3245 |
| *Acetobacter sp.* | ATCC 21760 |
| *Alcaligenes faecalis* | ATCC 8750 |
| *Escherichia coli* | ATCC 11105 |
| *Escherichia coli* | ATCC 13281 |
| *Xanthomonas citri* | IFO 3835 |
| *Xanthomonas physalidicola* | IFO 13555 |
| *Gluconobacter liquefaciens* | ATCC 14835 |
| *Gluconobacter dioxyacetonicus* | IFO 3271 |
| *Comamonas terrigena* | IFO 12685 |
| *Corynebacterium tritici* | IFO 1216 |

| | |
|---|---|
| *Sarcina lutea* | ATCC 9341 |
| *Staphylococcus aureus* | IFO 3060 |
| *Spirillum methanorphum* | IFO 12012 |
| *Bacillus megaterium* | ATCC 14945 |
| *Pseudomonas melanogenum* | ATCC 17808 |
| *Pseudomonas aeruginosa* | IFO 3451 |
| *Flavobacterium sp.* | ATCC 21429 |
| *Brevibacterium cerinum* | ATCC 15112 |
| *Protaminobacter alboflavus* | IFO 13221 |
| *Proteus rettgeri* | ATCC 9250 |
| *Beneckea hyperoptica* | ATCC 15803 |
| *Mycoplana bullata* | IFO 13267 |
| *Mycoplana dimorpha* | IFO 13213 |
| *Rhodopseudomonas spheroides* | ATCC 21286 |

As a medium, an aqueous solution containing 1% meat extract, 1% peptone, 0.3% sodium chloride and 0.5% yeast extract and adjusted to a pH of 7.2 with 5N-NaOH is used. One loopful seed strain is inoculated into 30 ml of the seed medium in a 300 ml Erlenmeyer flask and culturing is carried out at a temperature of 30°C for 24 hours. Cell bodies obtained by centrifugation from the culture broth are washed with 5 ml of 0.9% saline solution and again recovered by centrifugation therefrom. The cell is suspended in 1/30M phosphate buffer (pH 7.0) in a concentration of 20 mg/ml for dry weight.

5—2. Preparation of a substrate solution

150 mg of (±)-cis-7-[(R)-2-phenyl-2-aminoacetamido]-1-azabicyclo-[4,2,0]oct-2-en-8-on-2-carboxylic acid prepared as in DE-A-2911787 is dissolved in 15 ml of 1/30M phosphate buffer (pH 7.0).

5—3. Enzyme reaction

0.5 ml of each cell suspension prepared as in Example 5—1 and 0.5 ml of the substrate solution are mixed and the mixture is allowed to react at a temperature of 30° for 20 hours.

5—4. Identification of the product

It is possible to assay diastereoisomers of (±)-cis-7-[(R)-2-phenyl-2-aminoacetamido]-1-azabicyclo[4,2,0]oct-2-en-8-on-2-carboxylic acid by high speed liquid chromatography as described in DE-A-2911787. In this Example, diastereoisomer is quantitatively determined by such method. Quantitative determination of 7 -amino-1-azabicyclo[4,2,0]oct-2-en-8-on-2-carboxylic acid produced in the above 5—3 is possible in the same manner.

As apparent from Table 2, in the reaction solution, a more popular diastereoisomer, i.e., (—)-cis-7-[(R)-2-phenyl-2-aminoacetamido]-1-azabicyclo[4,2,0]oct-2-en-8-on-2-carboxylic acid remains unchanged and a less polar isomer, i.e., (+)-cis-7-[(R)-2-phenyl-2-aminoacetamido]-1-azabicyclo[4,2,0]-oct-2-en-8-on-2-carboxylic acid is decreased. Corresponding to the decrease, a peak of (+)-cis-7 -amino-1-azabicyclo[4,2,0]oct-2-en-8-on-2-carboxylic acid is formed. The imbalance of the decrease of (+)-cis-7-[(R)-2-phenyl-2-aminoacetamido]-1-azabicyclo[4,2,0]oct-2-en-8-on-2-carboxylic acid and the formation of (+)-cis-7 -amino-1-azabicyclo[4,2,0]oct-2-en-8-on-2-carboxylic acid depends on the presence of β-lactamase in the reaction system.

Enzymes of all microorganisms in Table 2 have an ability of producing optically active compound having the absolute configuration (6R, 7S) by selectively deacylating cephalosporin analogs.

TABLE 2

| Microorganism | The amount of* 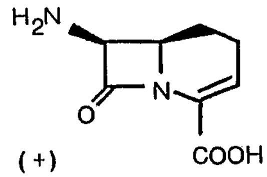 (+) produced (mg) | The amount of** 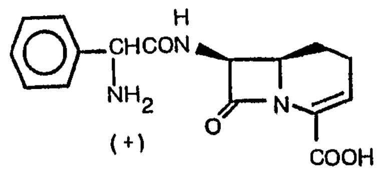 (+) decreased. (mg) | The amount of 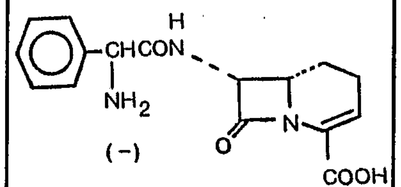 (-) decreased. (mg) |
|---|---|---|---|
| Aeromonas hydrophila IFO 12634 | 0.2 | 2.3 | 0 |
| Achromobacter aceris IFO 3320 | 0.4 | 2.0 | 0 |
| Arthrobacter simplex ATCC 15799 | 0.2 | 0.8 | 0 |
| Acetobacter aurantius IFO 3245 | 0.3 | 1.1 | 0 |
| Acetobacter sp. ATCC 21760 | 0.3 | 2.5 | 0 |
| Alcaligenes faecalis ATCC 8750 | 0.4 | 1.1 | 0 |
| Escherichia coli ATCC 11105 | 0.5 | 1.5 | 0 |
| Escherichia coli ATCC 13281 | 0.3 | 1.0 | 0 |
| Xanthomonas citri IFO 3835 | 0.9 | 2.5 | 0 |
| Xanthomonas physalidicola IFO 13555 | 1.3 | 2.5 | 0 |
| Beneckea hyperoptica ATCC 15803 | 1.1 | 2.4 | 0 |
| Gluconobacter liquefaciens ATCC 14835 | 0.8 | 2.4 | 0 |
| Gluconobacter dioxyacetonicus IFO 3271 | 1.2 | 2.4 | 0 |
| Comamonas terrigena IFO 12685 | 0.1 | 0.3 | 0 |

TABLE 2 (Continued)

| Microorganism | The amount of* 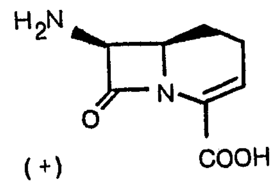 (+) produced (mg) | The amount of** 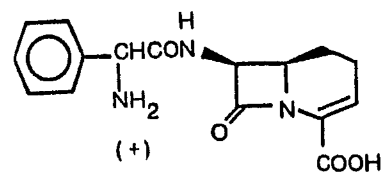 (+) decreased. (mg) | The amount of 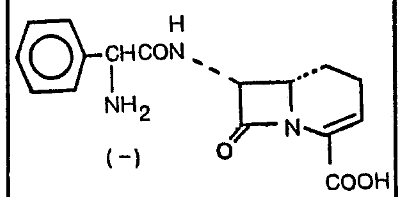 (-) decreased. (mg) |
|---|---|---|---|
| Corynebacterium tritici IFO 1216 | 0.2 | 0.4 | 0 |
| Sarcina lutea ATCC 9341 | 0.1 | 0.3 | 0 |
| Staphylococcus aureus IFO 3060 | 0.1 | 0.3 | 0 |
| Spirillum methamorphum IFO 12012 | 0.2 | 1.0 | 0 |
| Bacillus megaterium ATCC 14945 | 0.1 | 0.8 | 0 |
| Pseudomonas melanogenum ATCC 17808 | 1.3 | 2.5 | 0 |
| Pseudomonas aeruginosa IFO 3451 | 0.5 | 1.1 | 0 |
| Flavobacterium sp. ATCC 21429 | 0.5 | 1.5 | 0 |
| Brevibacterium cerinum ATCC 15112 | 0.4 | 0.8 | 0 |
| Protaminobacter alboflavus IFO 13221 | 1.2 | 2.5 | 0 |
| Proteus rettgeri ATCC 9250 | 0.1 | 0.2 | 0 |
| Mycoplana bullata IFO 13267 | 1.0 | 2.5 | 0 |
| Mycoplana dimorpha IFO 13213 | 1.3 | 2.5 | 0 |
| Rhodopseudomonas spheroides ATCC 21286 | 0.3 | 0.6 | 0 |

* When the yield is 100%, 1.4 mg of the compound is produced.

** The initial concentration is 2.5 mg.

Example 6

Preparation of (+)-cis-7 -amino-1-azabicyclo[4,2,0]oct-2-en-8-on-2-carboxylic acid (Alternative method):

*Clostridium acetobutylicum* IFO 3346 is inoculated in 100 ml of Potato Dextrose Broth (product of DIFCO Lab.). Air in the fermentor is replaced with sterilized nitrogen gas and the fermentor is sealed. Culturing is carried out at a temperature of 30°C for 48 hours. After the cultivation, cells are recovered, washed with physiological saline solution and suspended in 2 ml of 1/30M potassium phosphate buffer (pH 7.0). 0.5 ml of the substrate solution prepared as in Example 5—2) and the cell suspension are combined and allowed to react at a temperature of 30°C for 20 hours. The reaction is monitored as in Example 5—4). (—)-cis-7-[(R)-2-phenyl-2-aminoacetamido]-1-azabicyclo[4,2,0]oct-2-en-8-on-2-carboxylic acid remains unchanged and only (+)-cis-7-[(R)-2-phenyl-2-aminoacetamido]-1-azabicyclo[4,2,0]oct-2-en-8-on-2-carboxylic acid is decreased. The amount of the decrease is 1.0 mg, producing 0.2 mg of (+)-7 -amino-1-azabicyclo[4,2,0]oct-2-en-8-on-2-carboxylic acid.

Example 7

Preparation of (—)-cis-7-amino-3-chloro-1-azabicyclo[4,2,0]oct-2-en-8-on-2-carboxylic acid:

7—1. Preparation of disrupted cell suspension

1) Cultivation of a microorganism having an ability of optically selective deacylation

As the seed strain, *Kluyvera citrophila* ATCC 21285 [Biological properties are described in J. General Applied Microbiology, *3*, 28—31 (1957)] is used.

As the seed medium, an aqueous solution containing 1% polypeptone, 1% yeast extract, 0.5% meat extract, 0.5% sodium glutamate and 0.25% sodium chloride and adjusted to a pH of 7.0 with 5N-NaOH is used. One loopful seed strain is inoculated into 10 ml of the seed medium in a 50 ml test tube and culturing is carried out at a temperature of 30°C for 24 hours. The whole of the seed broth is inoculated into 300 ml of the culture medium in a 2 l Erlenmeyer flask and culturing is carried out at a temperature of 30°C with shaking. The composition of the main culture medium is the same as that of the seed medium.

2) Preparation of disrupted cell suspension

After culturing for 24 hours, the culture broth is subjected to centrifugation to obtain cell bodies. The cells are washed twice with 50 ml of 0.9% saline solution and suspended at a concentration of 40 mg/ml by dry weight in 1/30M phosphate buffer solution (pH 8.0).

7—2. Preparation of a substrate solution

In this step, 200 mg of (±)-cis-7-phenylacetamido-3-chloro-1-azabicyclo[4,2,0]oct-2-en-8-on-2-carboxylic acid obtained as in Reference Example 7 below is added to 9 ml of 1/30M phosphate buffer (pH 8.0). Since the compound is not dissolved, 2N-NaOH is added in a small portion and the mixture is again adjusted to a pH of 8.0 to dissolve the compound. Finally, deionized water is added to make 10 ml of a solution.

7—3. Enzyme reaction

In this step, 10 ml of the disrupted cell suspension mentioned above is added to 10 ml of the substrate solution and enzyme reaction is carried out at a temperature of 40°C for 80 minutes. Time course of the reaction is illustrated in Table 3.

Table 3

| Reaction period (minutes) | The amount of Compound [I—1] produced (mg/ml) | Yield (Mol ratio, %) |
|---|---|---|
| 10 | 1.3 | 20 |
| 20 | 1.8 | 28 |
| 40 | 2.0 | 31 |
| 60 | 2.3 | 36 |
| 80 | 2.4 | 37 |

7—4. Isolation and Purification of the desired compound

After the completion of the reaction, cells are removed by centrifugation from the reaction solution. The supernatant liquid is concentrated under reduced pressure to make 5 ml of solution. The solution is charged on a column (1.75 cm width, 42 cm height) packed with Diaion HP—10. Elution is

carried out with deionized water. The desired compound is eluted in fractions from 90 ml to 120 ml. The fractions are concentrated under reduced pressure, to make 2 ml of solution and the solution is adjusted to pH 3.5 with 1N-hydrochloric acid to deposit crystals. The crystals are recovered by filtration, washed with a small amount of methanol and dried to obtain 38 mg of a white powder. Properties of the product are as follows.

IR(KBr)$\nu_{max}^{cm^{-1}}$ : 3200, 1800, 1790(sh), 1640(sh), 1630, 1555

NMR(100M $D_2O$-DSS)$\delta$: 4.47(1H,d,J=5.1Hz), 3.88(1H,m), 2.64(2H,m), 1.93(2H,m)

Optical rotation $[\alpha]_D^{25°} = -2.7°$ (c=0.24, 1M phosphate buffer pH 7.0)

## Reference Example 1

Preparation of (+)-cis-7 -[2-(2-amino-4-thiazolyl)-2-syn-methoxyiminoacetamido]-1-azabicyclo-[4,2,0]oct-2-en-8-on-2-carboxylic acid:

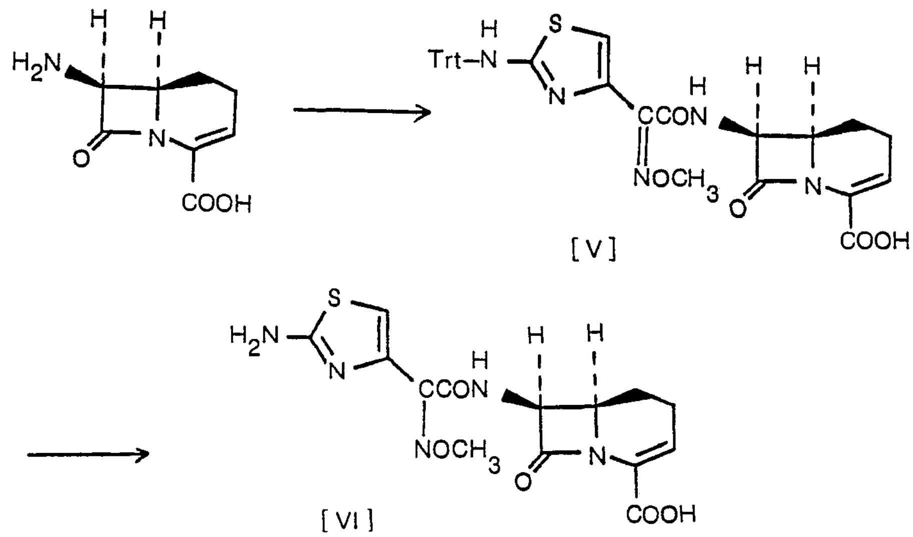

[V]

[VI]

In this Example, 131.3 mg (0.30 m mole) of 2-(2-tritylamino-4-thiazolyl)-2-syn-methoxyimino-acetic acid is dissolved in 1 ml of anhydrous dichloromethane and 4.1 $\mu l$ of triethylamine is added at a temperature of −20°C. Then, after adding 61.7 mg of phosphorus pentachloride, the mixture is allowe a temperature of −20°C. Then, after adding 61.7 mg of phosphorus pentachloride, the mixture is allowed to react a temperataure of −20°C for 30 minutes and concentrated under reduced pressure. The residue is dissolved in 1 ml of anhydrous tetrahydrofuran to make an acid chloride solution.

Separately, 40.2 mg (0.17 m mole) of the monohydrate of the hydrochloride of (+)-cis-7-amino-1-azabicyclo[4,2,0]oct-2-en-8-on-2-carboxylic acid obtained as in Example 1 is dissolved in a mixture of 1 ml of tetrahydrofuran and 1 ml of water and 116.2 $\mu l$ of triethylamine is added. The acid chloride solution prepared above is added dropwise to the solution with stirring under ice cooling and the mixture is allowed to react for one hour. Then, the mixture is adjusted to a pH of 2.0 with 5% hydrochloric acid and extracted 3 times with 10 ml of ethyl acetate. The ethyl acetate layers are washed with 10 ml of saturated saline solution, dried with saturated sodium sulfate and concentrated under reduced pressure to obtain 93 mg of a crude acyl compound. The product is dissolved in 10 ml of 50% acetic acid and stirred at a temperature of 50°C for 1.5 hours. The solution is cooled to room temperature and deposited white precipitate is removed by filtration. The filtrate is concentrated and the residue is dissolved in a small amount of dimethylsulfoxide. The solution is charged on a column packed with 10 ml of HP—10 and elution is carried out with water or a mixture of water and methanol (1:2). Fractions showing an Rf value of 0.3 by silica gel thin layer chromatography [plate: Merck® Art. 5719 (product of E. Merck & Co.), solvent : butanol : acetic acid : water = 4 : 1 : 1] are combined and concentrated under reduced pressure to obtain 13.5 mg (yield 22.4%) as white crystals. Properties of the product are as follows.

M.P.: 172°C (dec.)

$[\alpha]_D^{15°} = +32.6°$ (DMSO, c=0.5)

IR(KBr)$\nu_{max}^{cm^{-1}}$ : 1765, 1660, 1630, 1545

PMR(DMSO-$d_6$)$\delta$: 9.26(1H,d), 7.19(2H,s), 6.75(1H,s), 6.28(1H,t), 5.50(1H,d-d, J=8.9, 4.7Hz), 3.83(3H,s), 2.5—1.0(4H,m)

These values coincide well with those of the corresponding dl-compound. From the strong antimicrobial activity, absolute configuration of this compound is assumed to be (6R, 7S).

Reference Example 2

Preparation of (—)-cis-7β-[2-(2-amino-4-thiazolyl)-2-syn-methoxyiminoacetamido]-4α-methyl-1-azabicyclo[4,2,0]oct-2-en-8-on-2-carboxylic acid:

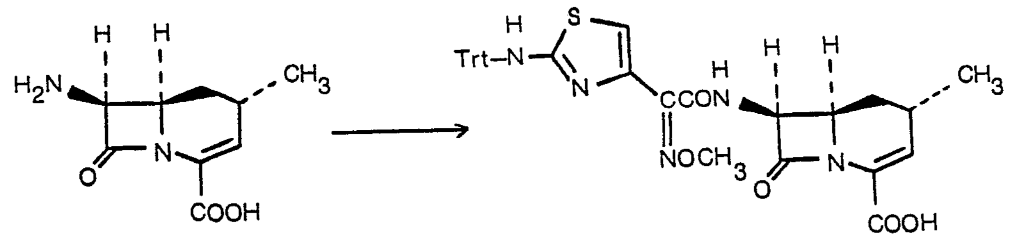

[VII]

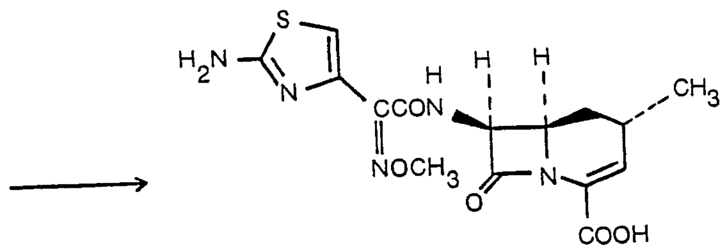

[VIII]

In this Example, 76 mg (0.17 m mole) of 2-(2-tritylamino-4-thiazolyl)-2-syn-methoxyiminoacetic acid is dissolved in 1.52 ml of anhydrous dichloromethane and 17.3 mg (0.17 m mole) of triethylamine is added at a temperature of —15°C. Then after adding 35.7 mg (0.17 m mole) of phosphorus pentachloride, the mixture is allowed to react with stirring at a temperature of —15°C for 30 minutes. The reaction mixture is concentrated under reduced pressure and the residue is dissolved in 2 ml of anhydrous tetrahydrofuran to make an acid chloride solution.

Separately, 28 mg (0.10 m mole) of the dihydrate of the potassium salt of (—)-cis-7β-amino-4α-methyl-1-azabicyclo[4,2,0]oct-2-en-8-on-2-carboxylic acid obtained as in Example 3 is dissolved in 1.5 ml of a mixture of tetrahydrofuran and water (1:1) and 36.3 mg (0.36 m mole) of triethylamine is added to make a homogeneous solution. To the solution is added to the acid chloride solution dropwise with stirring under ice cooling and the mixture is allowed to react for 45 minutes. Then, the reaction mixture is extracted four times with 3 ml of ethyl acetate. The ethyl acetate layer is washed with 5 ml of saturated saline solution, dried with saturated sodium sulfate, and concentrated under reduced pressure to obtain 107.1 mg of a crude acyl compound represented by the formula [VII]. The product is dissolved in 4.5 ml of 50% acetic acid and the solution is stirred at a temperature of 50 to 55°C for 45 minutes. After cooling to room temperature, the reaction solution is subjected to filtration to remove a deposited white precipitate and the cake is washed with 2 ml of 50% acetic acid. The washing and filtrate are combined and concentrated under reduced pressure. The residue is dissolved in a small amount of dimethylsulfoxide and charged on a column packed with 10 ml of HP—10. Elution is carried out with water and methanol (5:1 to 2:1). Fractions showing an Rf value at 0.54 by silica gel thin layer chromatography (the same conditions as mentioned above are used) are combined and concentrated under reduced pressure to obtain 12.9 mg (yield 23.8%) of the desired product as a white powder. Properties of the product are as follows.

M.P.: decompose at about 180°C

$[\alpha]_D^{15°} = -27°$ (DMSO, c=0.5)

IR(KBr)$\nu_{max}^{cm^{-1}}$: 1770, 1672, 1633, 1540

PMR(DMSO-$d_6$)δ: 9.26(1H,d,J=8.3Hz), 7.18(2H,s), 6.75(1H,s), 6.31(1H,d,J=5.1Hz), 5.51(1H,d-d,J=8.3, 5.0Hz), 3.83(3H,s), 1.67(2H,m), 1.07(3H,d,J=7.3Hz)

The values coincide well with those of the corresponding dl-compound. From the strong antimicrobial activity, absolute configuration of this compound is assumed to be (4S, 6R, 7S).

Reference Example 3

Antimicrobial activities of the compounds obtained in Reference Examples 1 and 2 are as follows. Heart Infusion Agar Dilution Method (pH 7.2) is used. The cephalosporin compound having the same acyl side chain corresponding to the dl-compound is used as a control.

A : The compound obtained in Reference Example 1.

A': The dl-compound corresponding to the compound obtained in Reference Example 1.

B : The compound obtained in Reference Example 2.
B′: The dl-compound corresponding to the compound obtained in Reference Example 2.
C : Cephalosporin compound represented by the following formula.

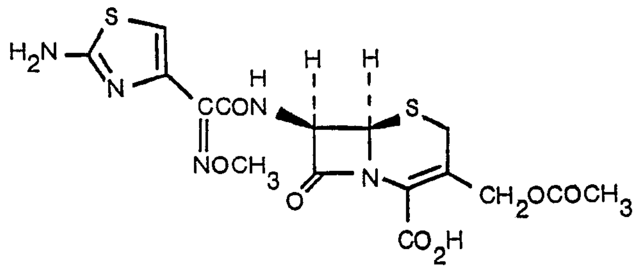

| Microorganism | MIC (μg/ml) | | | | |
|---|---|---|---|---|---|
| | A | A′ | B | B′ | C |
| Staphylococcus aureus 209–P | 3.12 | 12.5 | 1.56 | 6.25 | 0.78 |
| Staphylococcus aureus Smith | 6.25 | 25 | 6.25 | 12.5 | 1.56 |
| Staphylococcus epidermidis | 12.5 | 25 | 3.12 | 12.5 | 1.56 |
| Escherichia coli NIHJC–2 | 0.02 | 0.05 | 0.02 | 0.05 | 0.1 |
| Escherichia coli GN2411–5 | 0.01 | 0.05 | ≤0.01 | 0.02 | 0.05 |
| Escherichia coli Juhl | 0.02 | 0.1 | 0.02 | 0.05 | 0.05 |
| Klebsiella pneumoniae 8045 | ≤0.006 | ≤0.006 | ≤0.01 | 0.01 | ≤0.01 |
| Klebsiella pneumoniae Y–60 | 0.02 | 0.05 | 0.02 | 0.05 | 0.05 |
| Serratia marcescens T–26 | 0.2 | 0.78 | 0.4 | 0.78 | 0.78 |
| Serratia marcescens T–55 | 0.02 | 0.1 | 0.05 | 0.2 | 0.1 |
| Proteus mirabilis 1287 | 0.01 | 0.02 | ≤0.01 | 0.01 | 0.02 |
| Proteus vulgaris 6897 | ≤0.006 | 0.01 | ≤0.01 | 0.01 | ≤0.01 |
| Proteus morganii KY 4298 | 0.05 | 0.1 | 0.02 | 0.1 | 0.05 |
| Proteus rettgeri KY 4289 | ≤0.006 | ≤0.006 | ≤0.01 | 0.01 | ≤0.01 |
| Pseudomonas aeruginosa #1 | 6.25 | 25 | 25 | 50 | 6.25 |
| Pseudomonas aeruginosa 145 | 50 | 50 | 100 | >100 | 50 |
| Pseudomonas putida 264 | 0.1 | 0.4 | 0.05 | 0.2 | 0.1 |

Reference Example 4

Preparation of (±)-cis-7-azido-3-chloro-2-t-butyloxycarbonyl-1-azabicyclo[4,2,0]oct-2-en-8-one:

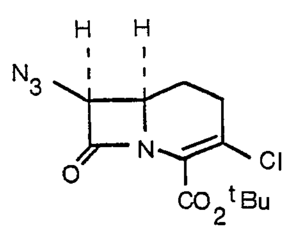

The present compound is prepared according to the following processes a), b), c) and d).

a) Preparation of (±)-cis-7-azido-3-phenylthio-1-azabicyclo[4,2,0]octane-8-on-2-carboxylic acid, t-butylester:

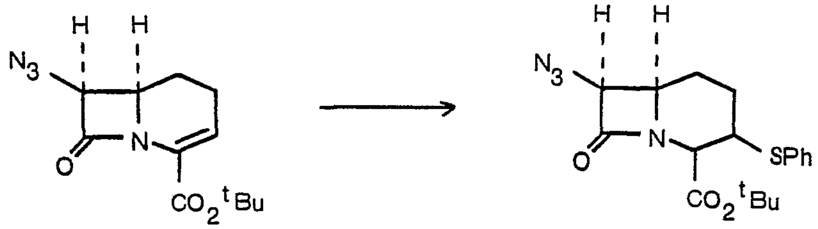

In this Example, 528 mg (2 m mole) of (±)-cis-7-azido-1-azabicyclo[4,2,0]oct-2-en-8-on-2-carboxylic acid, t-butylester prepared as in the method described in DE-A-2911786 is disolved in 15 ml of ahydrous benzene and 0.2 ml (2 m mole) of thiophenol and 0.2 ml (2 m mole) of piperidine are added. The mixture is stirred at room temperature for 2 hours. After the completion of reaction, the reaction solution is washed with 10% citric acid and saturated saline solution and dried with anhydrous sodium sulfate. The solvent is removed by distillation under reduced pressure and the obtained oily residue is purified by silica gel column chromatography using 30 g of silica gel and a solvent of ethyl acetate and n-hexane (1:4) to obtain 720 mg (96.3%) of the desired compound.
Melting point: 77.5—78.0°C
$IR\nu_{max}^{KBr}$ ($cm^{-1}$): 2110, 1765, 1745
NMR ($CDCl_3$)$\delta$: 7.27—7.60(5H,m), 4.78(1H,d,J=5Hz), 4.33(1H,s), 3.78—3.98(1H,m), 3.81(1H,s), 1.50—2.34(4H,m), 1.42(9H,s)

b) Preparation of (±)-cis-7-azido-3-phenylsulfinyl-1-azabicyclo[4,2,0]octane-8-on-2-carboxylic acid, t-butylester:

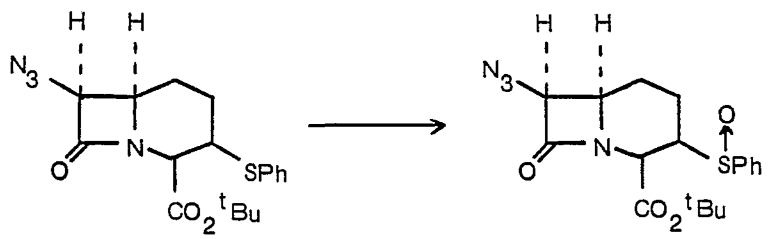

In this Example, 480 mg (1.28 m mole) of 3-phenylthio compound obtained as in Reference Example 4-a) is dissolved in 50 ml of anhydrous chloroform and 240 mg (1.41 m mole) of m-chloroperbenzoic acid under ice cooling. The mixture is allowed to react with stirring under ice cooling for 30 minutes. The reaction mixture is washed with saturated sodium bicarbonate and saturated sodium chloride and dried with anhydrous sodium sulfate. The solvent is removed by distillation under reduced pressure to obtain 500 mg (99.9%) of the desired compound.
Melting point: 95.5—96.5°C
$IR\nu_{max}^{KBr}$ ($cm^{-1}$): 2120, 2100, 1780, 1735, 1035
NMR ($CDCl_3$)$\delta$: 7.55—7.91(5H,m), 4.87(1H,d,J=4.6Hz), 4.05(1H,s), 3.90—4.10(1H,m), 3.10(1H,s), 1.70—2.84(4H,m), 1.30(9H,s)

c) Preparation of (±)-cis-7-azido-3-chloro-3-phenylsulfinyl-1-azabicyclo[4,2,0]octane-8-on-2-carboxylic acid, t-butylester:

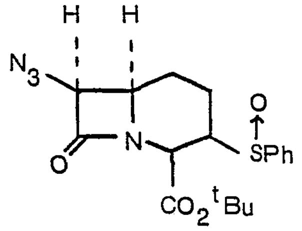

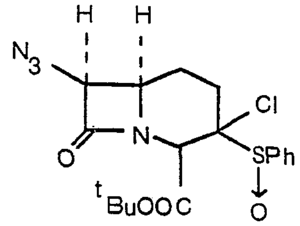

In this Example, 109 mg of sulfoxide compound obtained as in Reference Example 4-b) is dissolved in 1 ml of anhydrous methylene chloride and 23.5 mg (0.42 m mole) of calcium oxide is added. After adding 27 $\mu l$ (0.34 m mole) of sulfinyl chloride, the mixture is allowed to react with stirring under ice cooling for one hour. The reaction mixture is washed with 10% citric acid, saturated sodium bicarbonate and saturated sodium chloride solution and dried with anhydrous sodium sulfate. The

solvent is removed by distillation and the obtained oily residue is subjected to silica gel chromatography using 5 g of silica gel and a solvent of ethyl acetate and n-hexane (1:5) to obtain 66.5 mg (56.1%) of the purified desired oily compound.
IR$\nu_{max}^{CHCl_3}$ (cm$^{-1}$): 2120, 1770, 1735, 1055
NMR (CDCl$_3$)$\delta$: 7.53—8.00(5H,m), 4.90(1H,d,J=5Hz), 4.43(1H,s), 4.15—4.35(1H,m), 1.83—2.85(4H,m), 1.38(9H,s)

d) Preparation of (±)-cis-7-azido-3-chloro-1-azabicyclo[4,2,0]oct-2-en-8-on-2-carboxylic acid, t-butylester:

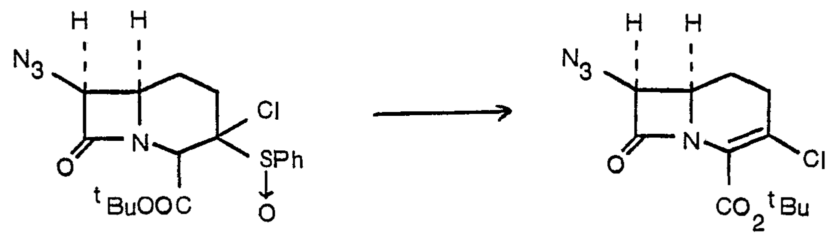

In this Example, 1.3 g (3.06 m mole) of 3-chloro-3-phenylsulfinyl compound obtained as in Reference Example 4-c) is heated under reflux with 100 ml of carbon tetrachloride for 6 hours. After the completion of reaction, the reaction solution is subjected to distillation under reduced pressure to remove the solvent. The obtained residue is subjected to purification by silica gel chromatography using 100 g of silica gel and a solvent of ethyl acetate and n-hexane (1:5) to obtain 596 mg (65.2%) of the desired compound.
Melting point: 96—97°C
IR$\nu_{max}^{KBr}$ (cm$^{-1}$): 2120, 1765, 1735, 1630
PMR (CDCl$_3$)$\delta$: 4.93(1H,d,J=5Hz), 3.72—3.92(1H,m), 2.56—2.70(2H,m), 1.86—2.32(2H,m), 1.55(9H,s),
CMR (CDCl$_3$)$\delta$: 127.7, 125.0

Reference Example 5

Preparation of (±)-cis-7-amino-3-chloro-1-azabicyclo[4,2,0]oct-2-en-8-on-2-carboxylic acid, t-butylester:

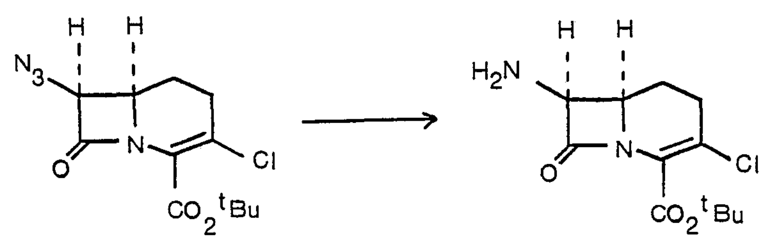

In this Example, 350 mg (1.17 m mole) of the azido compound obtained as in Reference Example 4 is dissolved in 20 ml of ethanol and 1.2 ml of 1N-hydrochloric acid and 70 mg of 10% palladium-carbon. Hydrogen gas is passed through the mixture at room temperature and atmospheric pressure for 3 hours and palladium-carbon is removed by filtration. The filtrate is concentrated under reduced pressure to obtain a solid. The solid is dissolved in water and washed with ether. After adding sodium bicarbonate to make weakly alkaline, the water layer is extracted with ethyl acetate. The ethyl acetate layer is washed with saturated saline solution and dried with anhydrous sodium sulfate. The solvent is removed by distillation under reduced pressure to obtain 218.4 mg (68.4%) of a powder of the desired compound.
Melting point: 102.5—104.5°C
IR$\nu_{max}^{KBr}$ (cm$^{-1}$): 1770, 1720, 1620
NMR (CDCl$_3$)$\delta$: 4.43(1H,d,J=5Hz), 3.52—3.90(1H,m), 2.52—2.72(2H,m), 2.22(2H,br), 1.87—2.17(2H,m), 1.55(9H,s)

Reference Example 6

Preparation of the trifluoroacetate of (±)-cis-7-amino-3-chloro-1-azabicyclo[4,2,0]oct-2-en-8-on-2-carboxylic acid:

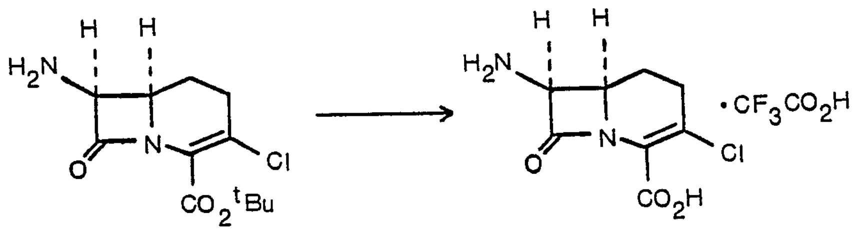

In this Example, 102.2 mg (0.31 m mole) of the amino-ester compound obtained as in Reference Example 5 and 1 ml of trifluoroacetic acid is added under ice cooling. The mixture is stirred at room temperature for 30 minutes. The solvent is removed by distillation under reduced pressure to obtain an oily residue. To the residue is added ether and the resulting powder is recovered by filtration. The powder is lyophilized to obtain 75.5 mg (60.9%) of the desired compound.

Melting point: 208—220°C (dec.)

$IR\nu_{max}^{KBr}$ ($cm^{-1}$): 1795, 1630

Reference Example 7

Preparation of (±)-cis-7-phenylacetamido-3-chloro-1-azabicyclo[4,2,0]oct-2-en-8-on-2-carboxylic acid:

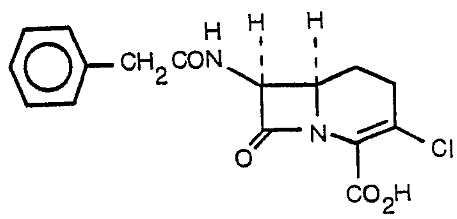

In this Example, 150 mg (0.45 m mole) of the trifluoroacetate of (±)-cis-7-amino-3-chloro-1-azabicyclo[4,2,0]oct-2-en-8-on-2-carboxylic acid prepared as in Reference Example 6 is dissolved in a mixture of 2 ml of water and 2 ml of acetone and 134 mg (1.5 m mole) of sodium bicarbonate is added to the solution to make the solution homogeneous. To the mixture is added 0.5 ml of phenylacetylchloride under cooling in one hour and the mixture is stirred for 3 hours. The reaction mixture is adjusted to a pH of 2 with 1N hydrochloric acid and extracted 5 times with 2 ml of ethyl acetate. The extract is concentrated under reduced pressure and dried to obtain 80 mg (55.0%) of the desired compound.

$IR\nu_{max}^{KBr}$ ($cm^{-1}$): 1790, 1705, 1630, 1560

NMR ($CD_3OD$)$\delta$: 7.29(5H,s), 5.36(1H,d,J=5Hz), 3.79—3.99(1H,m), 2.56—2.75(2H,m), 1.17—2.02(2H,m)

Reference Example 8

Preparation of (±)-cis-7-[(R)-2-phenyl-2-t-butyloxycarbonylamino]-3-chloro-1-azabicyclo[4,2,0]-oct-2-en-8-on-2-carboxylic acid, t-butylester:

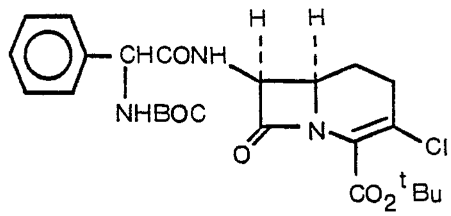

In this Example, 150 mg (0.55 m mole) of (±)-cis-7-amino-3-chloro-1-azabicyclo[4,2,0]oct-2-en-8-on-2-carboxylic acid, t-butylester obtained as in Reference Example 5 is dissolved in 3 ml of anhydrous methylene chloride. Separately, 166 mg (0.66 m mole) of N-t-butyloxycarbonyl-(R)-phenylglycine is dissolved in 5 ml of anhydrous tetrahydrofuran and the solution is cooled at a temperature of −15 to −10°C. Then, 0.66 ml of 1N-N-methylmorpholine in tetrahydrofuran and 0.66 ml of 1N-isobutyl chloroformate in tetrahydrofuran are added dropwise to the solution and the solution is stirred

at a temperature of −15 to −10°C for 20 minutes. Under the same temperature, the amine solution prepared above is added dropwise to the solution and the temperature is raised gradually to room temperature. The mixture stirred at room temperature overnight. After adding 10 ml of methylene chloride, the reaction mixture is washed with 10% citric acid solution, saturated sodium bicarbonate and saturated saline solution, dried with anhydrous sodium sulfate and concentrated under reduced pressure. The concentrate is subjected to silica gel chromatography using 20 g of silica gel (Wako-gel C 200, product of Wako Junyaku Co., Ltd.) and a solvent of ethyl acetate and n-hexane (1:5 by volume, the same shall apply hereinafter) to obtain 124 mg (yield 44.6%) of a mixture of the diastereoisomers of the desired compound. Properties of the product are as follows.

IR$\nu_{max}^{KBr}$ ($cm^{-1}$): 1780, 1730, 1680, 1655, 1550

NMR ($CDCl_3$)δ: 7.34(5/2H,s), 7.31(5/2H,s), 6.93(1H,m), 5.63(1H,m), 5.30(1/2H,dd,J=5.4, 6.8Hz), 5.11—5.22(3/2H,m), 3.76—3.91(1H,m), 2.33—2.66(2H,m), 1.52(9H,s), 1.41(9H,s), 0.92—1.97(2H,m)

Reference Example 9

Preparation of (6R, 7S) 7-(R)-phenylglycinamido-3-chloro-1-azabicyclo[4,2,0]oct-2-en-8-on-2-carboxylic acid (A) and (6S, 7R) 7-(R)-phenylglycinamido-3-chloro-1-azabicyclo[4,2,0]oct-2-en-8-on-2-carboxylic acid:

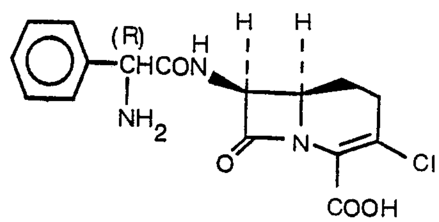

(A)

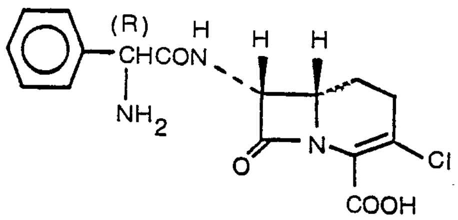

(B)

In this Example, to 128.4 mg (0.25 m mole) of (±)-cis-7-(2-phenyl-2-t-butyloxycarbonylamino-acetamido)-3-chloro-1-azabicyclo[4,2,0]oct-2-en-8-on-2-carboxylic acid, t-butylester obtained as in Reference Example 8 is added 1 ml of methylene chloride and 1 ml of trifluoroacetic acid under ice cooling and the mixture is stirred under ice cooling for 1.5 hours. The solvent is removed by distillation under reduced pressure and the obtained oily product is subjected to high speed liquid chromatography using Micro-Bondapack® C—18 (product of Waters, Co.) and a solvent of 7% methanol and 0.2N $KH_2PO_4$ to separate diastereoisomers. Each of the eluted fractions are concentrated under reduced pressure and the concentrates are subjected to desaltation with Diaion HP—10 and a solvent of methanol and water (1:1) to obtain 9.4 mg of a more polar isomer (B) and 7.6 mg of a less polar isomer (A).

Total yield 19.1%.

More polar isomer (B)

$[\alpha]_D^{21°}$: −75.8° (c=0.4, $H_2O$)

Melting point: more than 300°C (brown colored)

IR$\nu_{max}^{KBr}$ ($cm^{-1}$): 1765, 1700, 1550

NMR ($D_2O$)δ: 7.49(5H,s), 5.16(1H,d,J=4.7Hz). 5.05(1H,s), 3.78—4.03(1H,m), 2.53—2.67(2H,m), 1.26—2.09(2H,m)

Less polar isomer (A)

$[\alpha]_D^{21°}$: +34.0° (c=0.35, $H_2O$)

Melting point: more than 300°C (brown colored)

IR$\nu_{max}^{KBr}$ ($cm^{-1}$): 1770, 1700, 1620

NMR ($D_2O$)δ: 7.51(5H,s), 5.36(1H,d,J=4.6Hz), 5.19(1H,s), 3.83—4.00(1H,m), 2.41—2.56(2H,m), 1.49—1.76(1H,m), 1.14—1.45(1H,m)

The absolute structure of the isomer (A) is assigned as (6R, 7S) based on strong antibacterial activity of the less polar isomer (A) as mentioned below.

Reference Example 10

Preparation of (6R, 7S) 7-(R)-phenylglycinamido-3-chloro-1-azabicyclo[4,2,0]oct-2-en-8-on-2-carboxylic acid (alternative method):

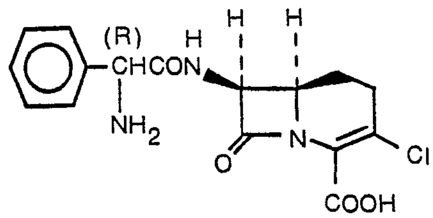

1)—1) Preparation of cell suspension

a) Cultivation of a microorganism having an ability of optically selective acylation.

As a seed strain, *Pseudomonas melanogenum* ATCC 17808 [Biological properties are described in Journal of the Agricultrual Chemical Society of Japan *37,* 71(1963)[ is used.

As the seed medium, an aqueous solution containing 1% polypeptone, 1% yeast extract, 0.5% meat extract, 0.5% sodium glutamate and 0.25% sodium chloride and adjusted to a pH of 7.0 with 5N-NaOH is used. One loopful seed strain is inoculated into 10 ml the seed medium in a 50 ml test tube and culturing is carried out at a temperature of 30°C for 24 hours. The whole amount of the seed medium is put into 300 ml of the culture medium in a 2 l Erlenmeyer flask and culturing is carried out at a temperature of 30°C. The composition of the culture medium is the same as that of the seed medium.

b) Preparation of cell suspension

After culturing for 24 hours, cell bodies are recovered from the culture broth by centrifugation and washed 2 times with 50 ml of 0.9% saline solution. The cells are suspended at a concentration of 20 mg/ml by dry weight in 1/30M phosphate buffer (pH 6.5).

10—2) Preparation of a substrate solution

200 mg of the trifluoroacetate of (±)-cis-7-amino-3-chloro-1-azabicyclo[4,2,0]oct-2-en-8-on-2-carboxylic acid (starting compound a) obtained as in Reference Example 6 and 800 mg of the hydrochloride of D-phenylglycinemethylester (starting compound b) are added to 9 ml of 1/30M potassium phosphate buffer (pH 6.5). 5N-KOH is added in a small portion and the mixture is again adjusted to a pH of 6.5 to dissolve the two starting compounds. Finally, deionized water is added to make 10 ml of solution.

10—3) Enzyme reaction

In the step, 10 ml of the cell suspension is added to 10 ml of the substrate solution and enzyme reaction is carried out at a temperature of 30°C for 1.5 hours. The reaction is monitored by high speed liquid chromatography using TRI ROTAR® (product of Nippon Bunko Co., Ltd.) and Prepack column Nucleosil ® 10C18 (product of Gaschro Kogyo Co., Ltd.). Elution is carried out with 7% methanol-0.2M $KH_2PO_4$ solution. Reaction reaches maximum in a yield of 90% to the starting compound a in 1.5 hours.

10—4) Isolation and Purification of the desired compound:

After the completion of reaction, cell bodies are removed from the reaction solution by centrifugation. The supernatant is concentrated under reduced pressure and charged on a column (1.6 cm width, 50 cm height) packed with Diaion HP—10. After adding 200 ml of deionized water, elution is carried out with 25% aqueous methanol solution. Then, the fractions containing the desired compound are concentrated under reduced pressure to make a 5 ml of concentrate. The concentrate is charged on a column (1.6 cm width, 64.5 cm height) packed with 130 ml of Sephadex-LH20 and elution is carried out with a solvent of water and methanol (50:50). The desired compound is eluted in fractions from 55 ml to 75 ml. The fractions are concentrated under reduced pressure and lyophilized to obtain 78 mg of a white powder. Properties of the product coincide with the less polar isomer (A) in Reference Example 9.

Antibacterial activities of the compounds obtained in Reference Examples 9 and 10 are shown in the following table. Heart Infusion Agar Dilution Method (pH 7.2) is used. Cefazolin is used as a control.

| Microorganism | MIC (μg/ml) Cefazolin | The less polar isomer (A) obtained in Reference Example 9 and the compound obtained in Reference Example 10 | The more polar isomer (B) obtained in Reference Example 9 |
|---|---|---|---|
| *Staphylococcus aureus* 209—P | ≤0.05 | 0.1 | 100 |
| *Staphylococcus aureus* Smith | 0.4 | 1.56 | >100 |
| *Staphylococcus epidermidis* | 0.78 | 1.56 | >100 |
| *Escherichia coli* NIHJC—2 | 1.56 | 1.56 | >100 |
| *Escherichia coli* Juhl | 1.56 | 1.56 | >100 |
| *Klebsiella pneumoniae* 8045 | 0.78 | 0.2 | 100 |
| *Klebsiella pneumoniae* Y—60 | 3.12 | 6.25 | >100 |
| *Serratia marcescens* T—26 | >100 | >100 | >100 |
| *Serratia marcescens* T—55 | 50 | 6.25 | >100 |
| *Proteus mirabilis* 1287 | 12.5 | 3.12 | >100 |

**Claims**

1. Optically active cephalosporin analogs represented by the general formula (I)

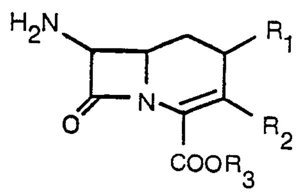

(I)

wherein $R_1$ represents a hydrogen or a straight or branched alkyl group, having 1 to 5 carbon atoms, $R_2$ represents a hydrogen or a halogen atom, $R_3$ represents a hydrogen or protective group of carboxylic acid, and the hydrogens at the 6- and 7-positions have cis configuration, and the pharmaceutically acceptable salts thereof.

2. The compound according to claim 1 wherein $R_1$ and $R_2$ are both hydrogen.

3. The compound according to claim 2 wherein $R_3$ is a hydrogen, that is, (±)-cis-7-amino-1-aza-bicyclo [4,2,0]oct-2-en-8-on-2-carboxylic acid.

4. The compound according to claim 1 wherein $R_1$ is a methyl group and $R_2$ is a hydrogen.

5. The compound according to claim 4 wherein $R_3$ is a hydrogen.

6. The compound according to claim 5 wherein the methyl group has the same configuration with the hydrogens at the 6- and 7-positions, that is, (—)-cis-7β-amino-4α-methyl-1-azabicyclo [4,2,0] oct-2-en-8-on-2-carboxylic acid.

7. The compound according to claim 1 wherein $R_1$ is a hydrogen and $R_2$ is a chlorine.

8. The compound according to claim 7 wherein $R_3$ is a hydrogen, that is, (—)-cis-7-amino-3-chloro-1-azabicyclo [4,2,0] oct-2-en-8-on-2-carboxylic acid.

9. A process for producing optically active compound represented by the general formula (I) according to claim 1 wherein $R_1$, $R_2$ and $R_3$ have the same meaning as in claim 1, and the hydrogens at the 6- and 7-positions have cis configuration, which comprises reacting a compound represented by the formula (III)

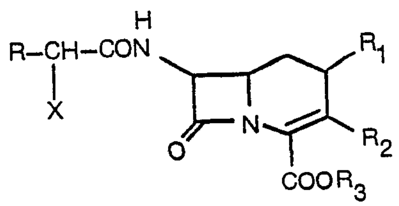

(III)

wherein R represents a substituted or unsubstituted unsaturated six-membered carbocyclic or five-membered heterocyclic group, wherein the substituent is represented by a hydroxy group, halogen atom, nitro group or methansulfonamide group, X represents a hydrogen, an amino group, a hydroxy group or an alkyl group having 1 to 5 carbon atoms, $R_1$, $R_2$ and $R_3$ have the same significance as defined above, and the hydrogens at the 6- and 7-positions has cis configuration with an enzyme capable of selective optical deacylation and thereafter recovering said optically active compound.

10. The process according to claim 9 wherein said enzyme is obtained from a microorganism belonging to the genus *Aeromonas, Achromobacter, Arthrobacter, Acetobacter, Alcaligenes, Escherichia, Xanthomonas, Kluyvera, Gluconobacter, Clostridium, Comamonas, Corynebacterium, Sarcina, Staphylococcus, Spirillum, Bacillus, Pseudomonas, Flavobacterium, Brevibacterium, Protaminobacter, Proteus, Beneckea, Micrococcus, Mycoplana* or *Rhodopseudomonas.*

11. The process according to claim 10 wherein said enzyme is provided to said reaction in the form of a purified enzyme solution, cell bodies recovered from a culture broth, a cell suspension, a disrupted cell suspension, a cell free extract, or a culture liquor of the microorganism.

12. Use of the compounds according to any of claims 1 to 8 for the preparation of pharmaceutical compositions with anti microbial activity.

**Patentansprüche**

(deutsche Übersetzung nach Artikel 97(5) EPÜ in Verbindung mit Regel 51 (4) EPÜ)

1. Optisch aktive Cephalosporinanaloga der allgemeinen Formel I

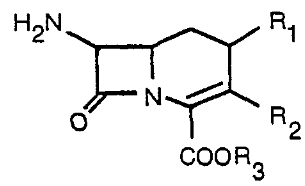

(I)

in der $R_1$ ein Wasserstoffatom oder einen gerad- oder verzweigkettigen Alkylrest mit 1 bis 5 Kohlenstoffatomen bedeutet, $R_2$ ein Wasserstoff- oder Halogenatom darstellt, $R_3$ ein Wasserstoff- oder eine Carbonsäureschutzgruppe bedeutet und in der die Wasserstoffatome in den 6- und 7-Stellungen die cis-Konfiguration haben, sowie ihre pharmazeutisch verträglichen Salze.

2. Verbindungen nach Anspruch 1, in denen $R_1$ und $R_2$ beide ein Wasserstoffatom bedeuten.

3. Verbindung nach Anspruch 2, dadurch gekennzeichnet, daß $R_3$ ein Wasserstoffatom bedeutet, nämlich (+)-cis-7-Amino-1-azabicyclo[4.2.0]oct-2-en-8-on-2-carbonsäure.

4. Verbindungen nach Anspruch 1, in denen $R_1$ eine Methylgruppe und $R_2$ ein Wasserstoffatom bedeutet.

5. Verbindung nach Anspruch 4, in der $R_3$ ein Wasserstoffatom bedeutet.

6. Verbindung nach Anspruch 5, in der die Methylgruppe die gleiche Konfiguration aufweist wie die Wasserstoffatome in den 6- und 7-Stellungen, nämlich (—)-cis-7$\beta$-Amino-4$\alpha$-methyl-1-azabicyclo-[4.2.0]oct-2-en-8-on-2-carbonsäure.

7. Verbindungen nach Anspruch 1, in denen $R_1$ ein Wasserstoffatom und $R_2$ ein Chloratom bedeutet.

8. Verbindung nach Anspruch 7, in der $R_3$ ein Wasserstoffatom bedeutet, nämlich (—)-cis-7-Amino-3-chlor-1-azabicyclo[4.2.0]oct-2-en-8-on-2-carbonsäure.

9. Verfahren zur Herstellung optisch aktiver Verbindungen der allgemeinen Formel (I) nach Anspruch 1, in denen $R_1$, $R_2$ und $R_3$ die in Anspruch 1 angegebene Bedeutung haben und die Wasserstoffatome in den 6- und 7-Stellungen die cis-Konfiguration aufweisen, dadurch gekennzeichnet, daß man eine Verbindung der allgemeinen Formel (III)

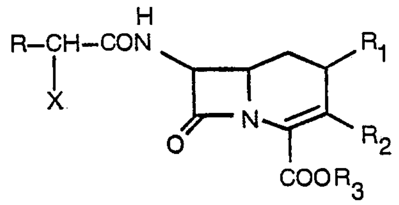

(III)

in der R einen substituierten oder unsubstituierten ungesättigten 6-gliedrigen carbocyclischen order 5-gliedrigen heterocyclischen Rest darstellt, wobei der Substituent eine Hydroxylgruppe, ein Halogenatom, eine Nitro- oder Methansulfonamidgruppe bedeutet, X ein Wasserstoffatom, eine Aminogruppe, eine Hydroxylgruppe oder einen Alkylrest mit 1 bis 5 Kohlenstoffatomen bedeutet, die Reste $R_1$, $R_2$ und $R_3$ die vorstehende Bedeutung haben und die Wasserstoffatome in den 6- und 7-Stellungen die cis-Konfiguration aufweisen, mit einem Enzym zur Umsetzung bringt, das die Fähigkeit zur selektiven optischen Entacylierung aufweist, und anschließend die genannte optisch aktive Verbindung gewinnt

10. Verfahren nach Anspruch 9, dadurch gekennzeichnet, daß man das genannte Enzym aus einem Mikroorganismus aus der Grupper der nachstehenden Gattungen erhält: Aeromonas, Achromobacter, Arthrobacter, Acetobacter, Alcaligenes, Escherichia, Xanthomonas, Kluyvera, Gluconobacter, Clostridium, Comamonas, Corynebacterium, Sarcina, Staphylococcus, Spirillum, Bacillus, Pseudomonas, Flavobacterium, Brevibacterium, Protaminobacter, Proteus, Beneckea, Micrococcus, Mycoplana oder Rhodopseudomonas.

11. Verfahren nach Anspruch 10, dadurch gekennzeichnet, daß man das Enzym für die genannte Umsetzung in Form einer gereinigten Enzymlösung, von aus einer Kulturbrühe gewonnenen Zellkörpern, einer Zellsuspension, einer unterbrochenen Zellsuspension, eines zellfreien Extraktes oder einer Kulturbrühe des Mikroorganismus einsetzt.

12. Verwendung der Verbindungen nach jedem der Ansprüche 1 bis 8 zur Herstellung von pharmazeutischen Zubereitungen mit antimikrobieller Wirksamkeit.

**Revendications**

1. Analogues de la céphalosporine optiquement actifs représentés par la formule générale (I)

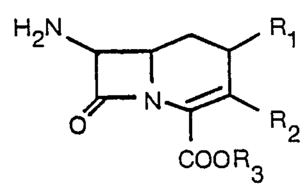

(I)

dans laquelle $R_1$ représente un hydrogène ou un groupe alkyle à chaîne droite ou ramifiée, ayant 1 à 5 atomes de carbone; $R_2$ représente un atome d'hydrogène ou d'halogène, $R_3$ représente un atome d'hydrogène ou un groupe protecteur d'acide carboxylique, et les hydrogènes sur les positions 6 et 7 ont la configuration cis, et leurs sels pharmaceutiquement acceptables.

2. Composé selon la revendication 1, dans lequel $R_1$ et $R_2$ sont tous les deux de l'hydrogène.

3. Composé selon la revendication 2, dans lequel $R_3$ est un hydrogène, c'est-à-dire, l'acide (+)-cis-7-amino-1-azabicyclo[4,2,0]oct-2-én-8-one-2-carboxylique.

4. Composé selon la revendication 1, dans lequel $R_1$ est un groupe méthyle, et $R_2$ est un hydrogène.

5. Composé selon la revendication 4, dans lequel $R_3$ est un hydrogène.

6. Composé selon la revendication 5, dans lequel le groupe méthyle a la même configuration avec les hydrogènes sur les positions 6 et 7, c'est-à-dire l'acide (—)-cis-7$\beta$-amino-4$\alpha$-méthyl-1-azabicyclo-[4,2,0]oct-2-én-8-one-2-carboxylique.

7. Composé selon la revendication 1, dans lequel $R_1$ est un hydrogène, et $R_2$ est un chlore.

8. Composé selon la revendication 7, dans lequel $R_3$ est un hydrogène, c'est-à-dire, l'acide (—)-cis-7-amino-3-chloro-1-azabicyclo[4,2,0]oct-2-én-8-one-2-carboxylique.

9. Procédé de préparation d'un composé optiquement actif représenté par la formule générale (I) selon la revendication 1, dans laquelle $R_1$, $R_2$ et $R_3$ ont la même signification que dans la revendication 1, et les hydrogènes sur les positions 6 et 7 ont la configuration cis, qui consiste à faire réagir un composé représenté par la formule III

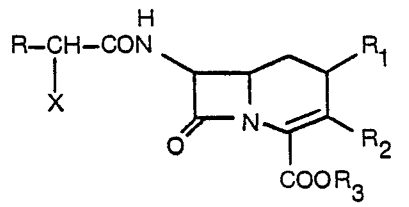

(III)

dans laquelle R représente un groupe carboxylique à six chaînons ou un groupe hétérocyclique à cinq chaînons, insaturés substitués ou non substitués, dans lequel le substituant est représenté par un groupe hydroxyle, un atome d'halogène, un groupe nitro ou un groupe méthanesulfamide; X représente un hydrogène, un groupe amino, un groupe hydroxyle ou un groupe alkyle ayant 1 à 5 atomes de carbone; $R_1$, $R_2$ et $R_3$ ont la même signification que définie cidessus, et les hydrogènes sur les positions 6 et 7 ont la configuration cis, avec une enzyme capable de désacylation optique sélective, puis à récupérer ledit composé optiquement actif.

10. Procédé selon la revendication 9, dans lequel ladite enzyme est obtenue à partir d'un micro-organisme appartenant au genre *Aeromonas, Achromobacter, Arthrobacter, Acetobacter, Alcaligenes, Escherichia, Xanthomonas, Kluyvera, Gluconobacter, Clostridium, Comamonas, Corynebacterium, Sarcina, Staphylococcus, Spirillum, Bacillus, Pseudomonas, Flavobacterium, Brevibacterium, Protaminobacter, Proteus, Beneckea, Micrococcus, Mycoplana* ou *Rhodopseudomonas.*

11. Procédé selon la revendication 10, dans lequel ladite enzyme est fournie à ladite réaction sous la forme d'une solution d'enzyme purifiée, de corps de cellule recueillis à partir d'un bouillon de culture, d'une suspension de cellules, d'une suspension de cellules rompues, d'un extrait exempt de cellules, ou d'un bouillon de culture de micro-organisme.

12. Utilisation des composés selon l'une quelconque des revendications 1 à 8 pour la préparation de compositions pharmaceutiques ayant une activité anti-microbienne.